# EUROPEAN PATENT SPECIFICATION

(11) **EP 0 543 215 B1**
(45) Date of publication and mention of the grant of the patent: **05.03.1997**
(21) Application number: 92118821.5
(22) Date of filing: 03.11.1992
(51) Int. Cl.: G01N 27/22, G01N 27/07, G01N 33/28

(54) **Sensor for determining the dielectric constant or the resistivity of a flowing liquid**
Sensor zur Bestimmung der dielektrischen Konstanten und des spezifischen Widerstands einer fliessenden Flüssigkeit
Capteur pour déterminer la constante diélectrique ou la résistivité d'un liquide en écoulement

(30) Priority: 19.11.1991 FR 9114218
(43) Date of publication of application: 26.05.1993
(73) Proprietor: SIEMENS AUTOMOTIVE S.A., 31036 Toulouse Cédex (FR)
(72) Inventor: Mouaici, Gérard, F-31500 Toulouse (FR)
(74) Representative: Fuchs, Franz-Josef, Dr.-Ing.

(56) References cited:
- EP-A- 0 379 644
- DE-A- 3 923 992
- FR-A- 2 409 389
- GB-A- 2 210 459
- US-A- 2 892 976

## Description

Sensor for determining the dielectric constant or the resistivity of a flowing liquid.

The present invention relates to a sensor for measuring the dielectric constant or the resistivity of a flowing liquid, which can be used especially for determining the ethanol content of a fuel mixture of hydrocarbons and ethanol.

It is known to mix, with hydrocarbons, relatively large amounts of alcohol such as methanol and/or ethanol for supplying internal-combustion engines. The engine settings, for optimum efficiency and minimum pollution, have to vary as a function of the composition, and it is known to slave these settings to this composition.

It is known that the dielectric constant of a hydrocarbon/alcohol mixture depends on its composition and varies approximately linearly with the alcohol content and appliances based on this observation have already been proposed.

DE-A-3,923,992 describes a sensor comprising a capacitor composed of a solid cylindrical central electrode and of a hollow cylindrical peripheral electrode which is coaxial with the central electrode. The two electrodes are placed in a chamber traversed by the mixture, which flows perpendicularly to the axis of the electrodes, the peripheral electrode exhibiting two longitudinal slots which are diametrically opposed in order to enable the mixture to pass between the electrodes. This sensor is quite complicated and, if it is desired to not introduce an undesirable pressure drop in the circuit of the mixture is to be avoided, it has to be quite voluminous.

Document DE-A-3,843,177 describes a sensor composed of a metal tube, through which the mixture flows and which constitutes an outer electrode of the capacitor, and of a solid cylinder held in an axial position by centring elements and which constitutes the central electrode. The tube forming the axial electrode is closed by insulating plugs. The connection of the central electrode traverses one of these plugs and the mixture enters the rube and leaves it via transverse passages. This device itself exhibits the drawback of being complicated, and bulky if an excessive pressure drop is to be avoided.

GB 2 210 459 discloses a conducting cell formed by two coaxial electrodes, the outer one being tubular and held around inner one by two spaced apart O-rings. The inner electrode comprises two bores extending from each end to beyond the O-ring forming liquid inlet and outlet. Radial passages are formed at the inner end of each bore, enabling liquid to flow in the space between electrodes. However, such a construction would also cause a drop of pressure accross the sensor, or lead to poor sensitivity if the space between electrodes is increased or to bulkiness if electrode diameter is increased.

The object of the present invention is to provide a sensor which is both less complicated, less expensive and less bulky, and having a pressure drop equal to the prior sensors.

In order to obtain this result, the invention provides a sensor for determining the dielectric constant or the resistivity of a flowing liquid, comprising a metal tube, inside which the liquid flows and which forms an outer electrode of a capacitor, and a cylindrical part coaxial with the said tube and which constitutes an inner electrode of the capacitor, the liquid flowing between the two electrodes, and insulating plugs located at the ends of the metal tube holding the two electrodes in a coaxial relative position, which exhibits the particular feature that the central electrode is itself tubular over its complete length and connected at one of its ends of a liquid inlet and at another end to a liquid outlet, the said central electrode exhibiting, towards each of the insulating plugs, openings enabling the liquid to pass into the space between the electrodes, and, between these openings, a means for limiting the direct passage of the liquid from one end of the said central electrode to the other.

The function of the means for limiting the direct passage of the liquid is to enable the liquid to be constantly replenished in the space between the two electrodes, at a rate appropriate to the circumstances. It is consequently easy to calculate the gap between the electrodes in order for the assembly not to create an additional pressure drop and yet, for the bulkiness of the device to be small, with an outer electrode of diameter only slightly greater than that of the inner electrode, which can be formed from a tube identical to the supply and outlet pipes of the liquid.

The operations necessary for making the device may be limited to forming the openings in the central electrode and installing the passage-limitation means, which can be performed very simply, either by forming a narrowed region on the tube using a press, or by force-fitting a ring as far as the chosen location. The electrical contacts can be established very simply, given that the two electrodes are easily accessible: clamping collars or soldered joints may be provided.

A very simple and low-cost structure is thus obtained.

In order to further simplify the construction, provision is advantageously made for the outer electrode to exhibit, close to at least one of its ends, an annular narrowed region into which a flexible extension of the insulating plug is snap-fitted, and the inner electrode exhibits, in the vicinity of the insulating plug, at least one enlarged region intended to prevent an axial displacement of the said plug and of the outer electrode.

The invention will now be explained in more detail with the aid of a practical example, illustrated by means of the drawings, among which:
- Figure 1 is a diagrammatic axial section of a device in accordance with the invention, and
- Figure 2 is a partial section similar to Figure 1, illustrating a variant of the means for limiting the direct passage of the liquid.
- Figure 3 is a detailed view illustrating the installation of an insulating plug.

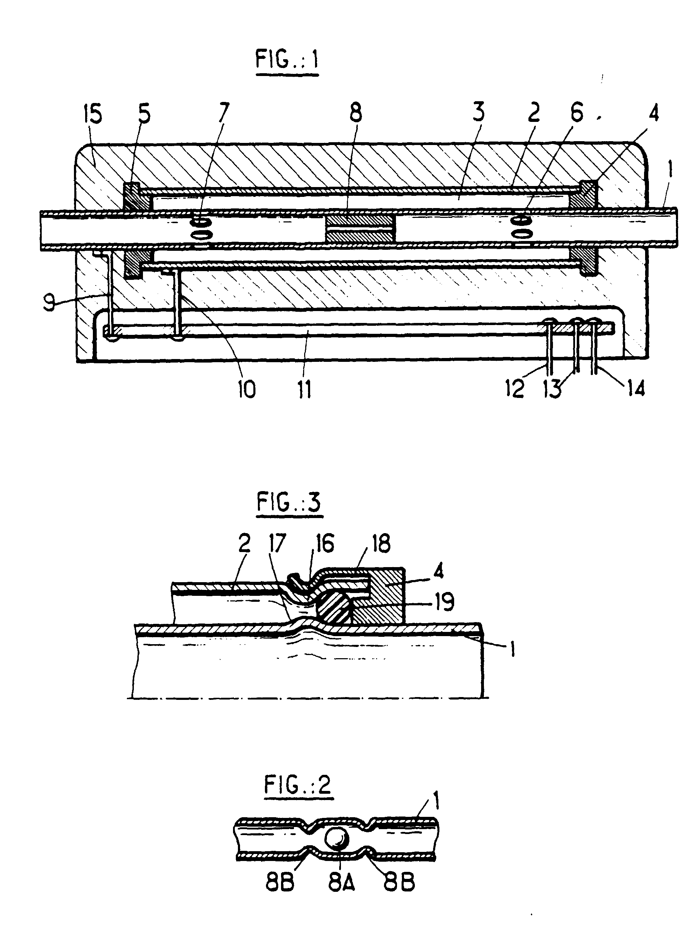

Figure 1 shows a metal tube 1, of the type which constitutes the usual petrol-supply pipes in a motor vehicle and which, here, additionally constitutes the inner electrode. A second metal tube 2, coaxial with the tube 1 and of slightly greater diameter, constitutes the outer electrode. The annular space 3 located between the two tubes is limited, at the ends of the tube 2, by two insulating and leaktight plugs 4 and 5. A series of radial perforations 6 and 7 traverses the inner tube 1 in the vicinity of the plugs 4, 5, but inside the space defined by these plugs, so as to bring the inside of the tube 1 into communication with the annular space 3. A ring 8, constituting a restriction, is force-fitted into the tube 1 between the two series of holes 6 and 7.

In another embodiment, illustrated by Figure 2, the ring 8 can be replaced by a ball 8A inserted into the tube 1 with the necessary clearance, in order to constitute the desired restriction. This ball can be held in place between two narrowed regions 8B in the tube. At least the narrowed region 8B located downstream of the ball, in the flow direction of the liquid, must not be circular so as not to constitute a valve element.

The tube 1 is connected, at one of its ends, to a fuel-inlet pipe, not shown, and at the opposite end, to a pipe for conveying fuel to an engine, these components also not being shown. It will be understood that the liquid fuel flow is split, inside the device, into two fractions, one continues directly through the restriction 8 while the other skirts around this restriction by virtue of the perforations 6 and 7 and of the annular chamber 3.

It is also understood that, if the restriction 8 is insufficiently effective, the major portion of the flow will traverse it and the replenishment of the liquid in the annular space 3 will be slow. On the contrary, if the restriction 8 is too pronounced, according to the diameter of the tube 2, the device will create an undesirable pressure drop in the fuel circuit, or alternatively the whole apparatus will be more bulky than necessary.

The connection strips 9 and 10 are welded, one to inner tube 1 beyond one of the plugs 4, 5, and the other to the outer tube 2. These connection blades connect the two tubes to an electronic circuit board 11 which is intended for shaping the signals corresponding to the variations in alcohol content of the fuel. The corresponding electronic circuits are within the scope of the person practised in the art, and are not described here. Connections 12, 13, 14 connect the board 11 outside the device. The whole device is housed in a protective case 15.

Figure 2 is a detailed view showing the structure of an insulating plug 4. The tube 2 exhibits, in the vicinity of one of its ends, an annular narrowed region 16. The inner tube 1 exhibits, in the same position, an enlarged, likewise annular, region 17. The insulating plug 4 includes a series of claws 18, moulded as one piece, and the dimension of which is calculated in order to snap fit, on the outside, into the narrowed region 16 when the plug 4 bears on the end of the tube 2. An O-ring seal 19 is wedged between the internal face of the narrowed region 16, the external face of the enlarged region 17, the internal and external walls of the outer 2 and inner 1 tube and the body of the plug 4, so as to ensure leaktightness.

Installation is designed to be extremely simple: after having slipped the outer tube 2 over the tube 1, the O-ring seal 19 is installed between these two tubes, which ensures the centring, and the plug 4 is pushed in, by a technique of the "punch" kind, until the claws 18 penetrate into the narrowed region 16. The opposite plug 5 is installed in the same manner.

In the case where the tube 1 exhibits, as is often the case, an enlarged region at its end with a view to its connection to another tube, the plug 4 can be made in two portions assembled together by screwing or otherwise, the seal preferably being embedded for better leaktightness.

The claws 18 may be replaced by a continuous flexible skirt of the same cross-section.

The apparatus which has been described is intended for monitoring the alcohol content of a fuel mixture, by measuring its dielectric constant. The persons skilled in the art will understand that it could also be used for determining the composition variations of a liquid by determination of its electrical resistivity.

In order to improve the accuracy of the composition determinations, it is possible to add to the device means for determining the temperature of the flowing liquid. In order to do this, for example, one of the connection blades 9, 10 can be folded back, in the vicinity of the corresponding tube 1, 2, in order to form a small tube inside which a thermistor or a thermocouple welded joint will be housed. A determination of the temperature is thus obtained at the actual location where the other parameter, the dielectric constant or resistivity, is determined.

## Claims

1. Sensor for determining the dielectric constant or the resistivity of a flowing liquid, comprising a metal tube (2), inside which the liquid flows and which forms an outer electrode of a capacitor, and a cylindrical part coaxial with the said tube and which constitutes an inner electrode (1) of the capacitor, the liquid flowing between the two electrodes, and insulating plugs (4, 5) located at the ends of the metal tube holding the two electrodes in a coaxial relative position, the central electrode (1) being connected at one of its ends to a liquid inlet and at another end to a liquid outlet, and exhibiting, towards each of the insulating plugs, openings (6, 7) enabling the liquid to pass into the space between the electrodes, characterized in that the said central electrode is tubular over its complete length and comprises between these openings, a means (8) for limiting the direct passage of the liquid from one end of the said central electrode to the other.

2. Sensor according the Claim 1, characterised in that the means for limiting the direct passage of the liquid is constituted by a narrowed region formed on the central electrode.

3. Sensor according to Claim 1, characterised in that the means for limiting the direct passage of the liquid is constituted by a ring (8) force-fitted into the said electrode.

4. Sensor according to Claim 1, characterised in that the means for limiting the direct passage of liquid is constituted by a ball (8A) inserted into the said electrode with the necessary clearance in order to constitute the desired restriction.

5. Sensor according to one of Claims 1 to 4, characterised in that the outer electrode exhibits, close to at least one of its ends, an annular narrowed region (16) into which a flexible extension (18) of the insulating plug is snapped-fitted and the inner electrode exhibits, in the vicinity of the insulating plug, at least one enlarged region (17) intended to prevent an axial displacement of the said plug and of the outer electrode.

## Patentansprüche

1. Sensor zum Bestimmen der dielektrischen Konstanten oder des Widerstandes einer strömenden Flüssigkeit, mit einem metallischen Rohr (2), in dem die Flüssigkeit strömt und die eine äußere Elektrode eines Kondensators bildet, und einem zu dem Rohr koaxialen zylindrischen Teil, das eine innere Elektrode (1) des Kondensators bildet, wobei die Flüssigkeit zwischen den beiden Elektroden strömt, und isolierenden Stopfen (4,5), die an den Enden des metallischen Rohres angeordnet sind und hierbei die beiden Elektroden in koaxialer Lage relativ zueinander halten, wobei die zentrale Elektrode (1) an einem ihrer Enden mit einem Flüssigkeitseinlaß und an einem anderen Ende mit einem Flüssigkeitsauslaß verbunden ist und in Richtung auf die isolierenden Stopfen Öffnungen (6,7) aufweist, durch die Flüssigkeit in den Raum zwischen den Elektroden strömen kann, dadurch gekennzeichnet, daß die zentrale Elektrode über ihrer gesamten Länge rohrförmig ist und zwischen diesen Öffnungen ein Mittel (8) zum Drosseln der Flüssigkeitsströmung von einem Ende der zentralen Elektrode zum anderen aufweist.

2. Sensor nach Anspruch 1, dadurch gekennzeichnet, daß das Mittel zum Drosseln der Flüssigkeitsströmung von einem verengten Bereich gebildet wird, der an der zentralen Elektrode vorgesehen ist.

3. Sensor nach Anspruch 1, dadurch gekennzeichnet, daß das Mittel zum Drosseln der Flüssigkeitsströmung von einem Ring (8) gebildet wird, der mit Preßsitz in der Elektrode angeordnet ist.

4. Sensor nach Anspruch 1, dadurch gekennzeichnet, daß das Mittel zum Drosseln der Flüssigkeitsströmung von einer Kugel (8A) gebildet wird, die in die Elektrode mit dem erforderlichen Spielraum zum Bilden der erwünschten Drosselung eingesetzt ist.

5. Sensor nach einem der Ansprüche 1 bis 4, dadurch gekennzeichnet, daß die äußere Elektrode in der Nähe zumindest einer ihrer Enden einen ringförmigen verengten Bereich (16) aufweist, in den eine flexible Verlängerung (18) des isolierenden Stopfens mit Schnappsitz eingesetzt ist, und daß die innere Elektrode in der Nähe des isolierenden Stopfens mindestens einen verbreiterten Bereich (17) aufweist, der dazu dient, eine axiale Verschiebung des Stopfens sowie der äußeren Elektrode zu verhindern.

## Revendications

1. Capteur pour déterminer la constante diélectrique ou la résistivité d'un liquide en circulation, comprenant un tube métallique (2) à l'intérieur duquel circule le liquide et qui forme une électrode extérieure d'un condensateur, et une pièce cylindrique coaxiale audit tube, et qui constitue une électrode intérieure (1) du condensateur, le liquide circulant entre les deux électrodes, des bouchons isolants (4, 5), situés aux extrémités du tube métallique assurant le maintien des deux électrodes dans une position relative coaxiale, une des extrémités de l'électrode centrale étant tubulaire et reliée à une arrivée de liquide, l'autre extrémité de l'électrode centrale étant tubulaire et raccordée à une sortie de liquide, ladite électrode centrale présentant, vers chacun des bouchons isolants, des ouvertures (6, 7) permettant le passage du liquide dans l'espace entre les électrodes, caractérisé en ce que l'électrode centrale est tubulaire d'une extrémité à l'autre et comprend, entre lesdites ouvertures, un moyen (8) pour limiter le passage direct du liquide d'une extrémité à l'autre de ladite électrode centrale.

2. Capteur selon la revendication 1, caractérisé en ce que le moyen pour limiter le passage direct du liquide est constitué par un rétreint formé sur l'électrode centrale.

3. Capteur selon la revendication 1, caractérisé en ce que le moyen pour limiter le passage direct du liquide est constitué par une bague (8) enfoncée à force dans ladite électrode.

4. Capteur selon la revendication 1, caractérisé en ce que le moyen pour limiter le passage direct de liquide est constitué par une bille (8A) introduite dans ladite électrode avec le jeu nécessaire pour constituer l'étranglement désiré.

5. Capteur selon l'une des revendications 1 à 4, caractérisé en ce que l'électrode extérieure présente, près d'au moins une de ses extrémités, un rétreint annulaire (16) dans lequel vient s'enclencher un prolongement flexible (18) du bouchon isolant, et l'électrode intérieure présente, à proximité du bouchon isolant, au moins un élargissement (17) destiné à empêcher un déplacement axial dudit bouchon et de l'électrode extérieure.
